(19) Europäisches Patentamt
European Patent Office
Office européen des brevets

(11) Publication number: **0 425 754 A1**

# (12) EUROPEAN PATENT APPLICATION

(21) Application number: **90101924.0**

(51) Int. Cl.[5]: **A61B 5/00**, A61B 5/0404

(22) Date of filing: **31.01.90**

The title of the invention has been amended (Guidelines for Examination in the EPO, A-III, 7.3).

(30) Priority: **23.10.89 JP 123851/89 U**

(43) Date of publication of application:
**08.05.91 Bulletin 91/19**

(84) Designated Contracting States:
**DE FR GB IT**

(71) Applicant: **FUKUDA DENSHI CO., LTD.**
**39-4, Hongo 3-chome Bunkyo-ku**
**Tokyo 113(JP)**

(72) Inventor: **Muraki, Yoshiya, c/o Fukuda Denshi Co., Ltd.**
**Hongo enterprice place, 2-35-8, Hongo, Bunkyo-ku**
**Tokyo(JP)**
Inventor: **Koike, Hiroyuki, c/o Fukuda Denshi Co., Ltd.**
**Hongo enterprice place, 2-35-8, Hongo, Bunkyo-ku**
**Tokyo(JP)**

(74) Representative: **Behn, Klaus, Dipl.-Ing.**
**Patentanwalt Lindenberg 34**
**W-8134 Pöcking bei München(DE)**

## (54) Portable apparatus for alerting a physician.

(57) A portable apparatus (1,100) comprising a radio receiver, a liquid crystal display screen (2) and alarm means (8,10) and carried by medical personnel by means of a belt clip (3) is adapted for monitoring physiological signals (5), in particular electrocardiogram signals, transmitted from one or more patients. In response to a suddenly changing condition of a patient, signal waveforms (E) are displayed and an alarm may be energised.

Fig. 1

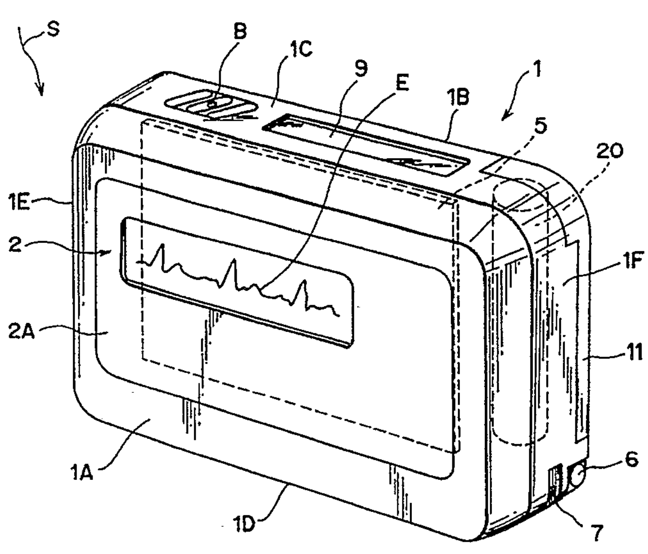


EP 0 425 754 A1

# APPARATUS FOR CALLING A DOCTOR UP BY RADIO SO AS TO GIVE AN ALARM FOR A PATIENT

## BACKGROUND OF THE INVENTION

### 1. Field of the Invention

The present invention relates to an apparatus for calling a doctor up by radio so as to give an alarm for a patient.

More particularly, it relates to an apparatus for calling a doctor up by radio so as to give an alarm for a patient, in which the wave form of the electrocardiogram signal of the patient is displayed.

### 2. Description of the Related Art

Geneally, in a hospital, a doctor has a portable receiver for calling up by radio so as to stand by to be called up when the condition of a patient has changed suddenly.

For example, it is usual that a few doctors keep watch on the condition of many patient with heart disease during the night in a hospital.

Hence, when the doctor gives his full time to one patient, he cannot know that the condition of another patient has changed suddenly.

In order to avoid such an inconvenience, the doctor carries always about the portable receiver so as to call up in the case that an alarm for a patient will be given.

A conventional apparatus for calling a doctor up by radio, has a display portion wherein messages are only displayed.

For example, a message "PATIENT ALARM NO = 5" is displayed.

A doctor, who has seen the message, thinks that the condition of the fifth patient has changed suddenly, and accordingly, he goes fast to and takes action for this patient.

However, the above message in "PATENT ALARM NO = 5" has been displayed, by means that a system for monitorring a patient setted in a hospital judges mechanically the condition of the patient.

Hence, though the patient, turning over merely in bed makes to change the direction of his heart, whereby the wave or of his electrocardiogram changes, the system judges sometimes that the condition of the patient has been changed suddenly.

The result is occasionally that the above message is displyed.

And, though electrodes fitted to the breast of the patient are taken off, thereby the wave form of his electorcardiogram changes, the system judges sometimes that the condition of the patient has been changed suddenly.

The result is occasionally that the above message is displayed.

These two cases are instances that an erroneous judgement of the system makes to display the above patient alarm message .

Hence, although the doctor has gone fast to the patient, there is mostly no need to take any action for the patient.

That is to say, since the doctor cannot judge whether or not the condition of the patient has been changed suddenly, it is found that he should take action for his patient owing to a mistaken message .

## SUMMARY OF THE INVENTION

An object of the present invention is to make a doctor to judge precisely with an apparatus for calling up by radio whether or not the condition of a patient has been changed suddenly.

The above-mentioned object can be achieved by an apparatus for calling a doctor up by radio so as to give an alarm a patient, which recerives a living body information signal transmitted through a transmitter and knows to a doctor with buzzer sound or vibration that the condition of the patient of the living body information signal has been changed suddenly, comprising a portable casing having a front plate, a rear plate, a top plate, a bottom plate, a left plate, and aright plate, said casing being provided with a circuit plate, on which circuit plate an electric circuit is mounted so as to receive and treat the living body information signals, and a display portion being furnished on said casing and being connected with said electric circuit, on which display portion the wave form of at least electrocardiogram signal of the living body signals is displayed.

## BRIEF DESCRIPTION OF THE DRAWINGS

Further features and advantages of the present invention will be apparent from the ensuring dencription with reference to the accompanying drawings, wherein:

Fig. 1 is a whole drawing of an embodiment of the present invention;

Fig. 2A to 2E are detailed drawings of the embodiment of the present invention; and

Fig. 3A to 3C are explanatory drawings of the operation of the present invention; and

Fig. 4 is a drawing of an application of the present invention.

## DESCRIPTION OF THE PREFERRED EMBODIMENTS

Figure 1 is a whole drawing of an embodiment of the present invention, wherein reference numeral 1 shows casing, 2 a display portion, 5 a circuit plate, 6 a monting pin, 7 a bar, 8 an opening, and 9 a groove.

The casing 1 has wholly the shape of a rectangular parallelepiped, and is formed by insulator, for example, plastics.

The casing 1 is so small that it has a width of 105mm, a hight of 74.5 mm, and a thickness of 30 mm.

The casing 1 has a circuit plate 5 within, on which circuit plate 5 an electric circuit is loaded so as to receive and treat a living body information signals S transmitted by radio from a transmitter 200 (see Fig, 4).

The above electric circuit can be operated, for example , by an alkali-manganese dry cell 20, which dry cell 20 has a 3 unit size and electromotive force of 1.5 V.

A display portion 2 is mounted at the upper area on the front plate 1A of the casing 1, which display portion 2 is formed by liquid crystal, for example, and is protected by a protecting plate 2A with tranparency and made of plastics.

On the display portion 2, the wave form E of a patient's electrocardiogram, of many living body information signals S, may be at least displayed, in case of the patient alarm , that is to say, when the condition of the patient has been changed suddenly.

On the display portion 2, except for the above wave form E, messages composed of English or numeral characters, for example the message "PATIENT ALARM NO = 5" are also displayed as in the prior art.

Light may be emitted to the back surface of the display portion 2 so as to be able to see easily the wave form E of the patient's electrocardiogram or messages in the dark place.

Figures 2A to 2E are detailed drawings of an embodiment of the present invention.

Fig 2A is a front view, Fig. 2B a top view, Fig. 2C a rear view, Fig. 2D a left side view, and Fig. 2E a right side view.

The opening 8 is formed on the top plate 1C of the casing 1, through which opening 8 buzzer sound is output so as to call a doctor up.

Moreover, on the top plate 1C of the casing 1, the groove 9 is formed, in which groove 9 a stamp tape may be stickked.

Means for calling a doctor up may be not only buzzer sound, but also vibration as described heinafter, which both are exchangeable each other.

A rear plate 1B is fixed, with screws 13, 14, 15 and 1 6, to the casing 1.

A belt clip 3 is mounted pivotally on the middle area of the rear plate 1B, a place 17 for skicking label of an owner's name etc. is formed on the left area of the same plate 1B, and a battery cover 11 is setted on the right area of the same plate 1B.

The battery cover 11 may be mounted and demounted with a claw 12 for locking, on which battery cover 11 projections 11 A and 11B are formed so as to operate easily it.

The belt clip 3 has a first member 3A and a second member 3B (see Fig. 2D and 2E), which members 3A and 3B are energized with spring so as to hold a belt 30 (see Fig. 3A).

The second member 3B is mounted pirotally on the casing 1, through a mounting portion 3C (see Fig. 3B).

The belt clip 3 may be taken off from the casing 1, by means of pulling the mounting pin 6 to the right direction (see Fig. 2C), thereafter a stand plate 4 may be mounted instead of the belt clip 3.

A receiving lamp 10 is installed on the left plate 1F of the casing 1, from the lower area thereof through the bottom plate 1D, which receiving lamp 10 goes on and off when the above-mentioned wave form E of electrocardiogram and other messages are received.

A first switch SW1, a second switch SW2, and a third switch SW3 are respectively furnished in order on the middle area of the left plate 1E.

The first switch SW1 is in the position of OFF, whereby an electric source in the casing 1 becomes off and the contents of data stored in a memory become to vanish.

And, the first switch SW1 is in the position of M as shown in Fig. 2D, whereby a doctor may be called up with vibration, and it is in the position of ON, whereby he may be called up with buzzer sound.

The second switch SW2 is one which switches on a light for irradiating the display portion2.

The functions of the third switch SW3 are as follows.

That is to say, it stops buzzer sound or vibration, changes a picture to be displayed on the display portion 2, as to the wave form E of the patien's electrocardiogram or messages, displays the contemts of data stored in the memory, and clears the contents displayed on the display portion 2 by keeping to be pushed, etc..

The above-mentioned mounting pin 6 and the bar 7 are furnished respectively, from the lower area of the right plate 1F through the bottom plate 1D.

The bar 7 is one round which a chain clip is wound so as to prevent the casing 1 from falling down when it is put in a pocket etc..

The operation of the present invention will be discribed hereinafter with reference to Figs. 3A to 3C.

The belt 30 is gripped with the first member 3A and second member 3B constituting the belt clip 3, whereby an apparatus for calling a doctor up by radio in accordance with the present invention may be mounted on the human body, as shown in Fig. 3A.

When the condition of a patient has been changed suddenly, buzzer sound or vibration calls a doctor up as well as the wave form E of the patient's electrocardiogram is displayed on the display portion 2.

The casing 1 is pivotted by 90 ° to the direction shown in an arrow A, whereby the wave form E can be observed clearly.

The third switch SW3 is pushed, whereby the name of the patient having the wave form E is displayed on the display portion 2.

Accordingly, the doctor can judge precisely whether or not the condition of the patient with the wave form E displayed has been changed suddenly.

When it is unnecessary to look at the display portion 2, the casing 1 is pivotted to the direction shown in an arrow B, whereby it is returned the original position, as sown in Fig. 3A.

The belt clip 3 may be demounted by moving the mounting pin 6 to the direction shown in the arrow C of Fig. 3B, whereby the stand plate 4 can be mounted instead of the belt clip 3, as shown in Fig. 3C.

The stand plate 4 has the effect that the wave form E shown on the display portion 2 can be observed by standing the apparatus in accordance with the present invention on a desk 40, for example, as shown in Fig. 3 C.

Figure 4 is a drawing of an application of the present invention, wherein reference numeral 100 is the apparatus in accordance with the present invention, 200 a transmitter, 300 a controller, 400 a private branch exchange (PBX), 500 a telephone, 500 a murse caller, 700 a monitor, and 800 an electrocardiogram (ECG) telemeter.

The transmitter 200 is an apparatus which modulates signal output from the controller 300 and transmits it to the apparatus 100 in accordance with the present invention as the living body information signals S.

The controller 300 is an apparatus which treats signal output from the PBX 400, the nurse caller 600, or, the monitor 700, and controls to connect these apparatuses with the transmitter 200 , smoothly.

The PBX 400 is an apparatus which exchanges to connect the telephone 500 with an extention or with an outside line.

The nurse caller 600 is an apparatus in which a nurse pushes the switch corresponding to the name of the specific patient, thereby the sudden change of the patient's condition is known to a doctor.

The monitor 700 is an apparatus which keeps watch on electrocardiogram etc. of a patient whose condition may be changed suddenly.

The ECG telemeter 800 is an apparatus which modulates electrocardiogram signal led out from the living body of a patient and transmits it to the monitor 700.

As apparenly from Fig. 4, the apparatus 100 in accordance with the present invention may be applied when a doctor will be called up by using, for example, the telephone 500, the nurse caller 600, and the monitor 700.

That is to say, the apparatus 100 may be linked with the telephone 500, the nurse caller 650, and the monitor 700, repectively.

The linkage with the telephone 500 is a personal call for calling one specific person up, or a group call for calling one specific group up etc..

The linkagewith the nurse caller 600 is an instance where a doctor may be called up by pushing the switch, as described hereinbefore, in the nurse caller 600.

The linkage with the monitor 700 is an instance where a doctor may be called up by displaying the wave form E of the specific patient's electrocardiogram signal on the display portion 2, as described already.

According to the present invention, even if the patient monitorring system outputs an erroneous patient alarm message, a doctor can guess roughly the condition of a patient by observing the wave from E of the patient's electrocardiogram signal displayed on the display portion 2.

Hence, the present invention has the effect that a doctor can judge precisely, with an apparatus for calling up by radio, whether or not the condition of a patient bas been ohanged suddenly.

## Claims

1. An apparatus for calling a doctor up by radio so as to give an alarm a patient, which recerives living body information signals transmitted through a transmitter and knows to a doctor with buzzer sound or vibration that the condition of the patient of the living body information signals has been changed suddenly, comprising;
a portable casing 1 having a front plate 1A, a rear plate 1B, a top plate 1C, a bottom plate 1D, a left plate 1E, and a right plate 1F, said casing 1 being provided with a circuit plate 5, on which circuit plate 5 an electric circuit is mounted so as to receive and treat the living body information signals S, and
a display portion 2 being furnished on said casing 1 and being connected with said electric circuit, on which display portion 2 the wave form E of at least electrocardiogram signal of the living body information signals S is displayed.

2. An apparatus for calling a doctor up by radio so as to give an alarm a patient according to claim 1, wherein said display portion 2 is furnished on said front plate 1A of said casing 1, and is formed by liquid crystal.

3. An apparatus for calling a doctor by radio so as to give an alarm a patient according to claim 1, wherein a belt clip 3 is mounted pivotally on said rear plate 1B of said casing 1.

4. An apparatus for colling a doctor up soas to give an alarm a patient according to claim 1, wherein a stand plate 4 is mounted pivotally on said rear plate 1B of said casing 1.

5. An apparatus for calling a doctor up so as to give an alarm a patient according to claim 1, wherein a belt clip 3 and a stand plate 4 are exchangedble together to said casing 1.

6. An apparatus for calling a doctor up so as to give an alarm a patient according to claim 1, wherein
a receiving lamp 10 is installed from the lower area of said left plate 1E throgh said bottom plate 1D, and a first switch SW1, a second switch SW2, and a third switch SW3 are furnished on the middle area of said left plate 1E.

7. An apparatus for calling a doctor up so as to give an alarm a patient according to claim 1, wherein a mounting pin 6 and a bar 7 are furnished from the lower area of said right plate 1F throgh said bottom plate 1D.

8. An apparatus for calling a doctor up so as to give an alarm a patient according to claim 1, wherein an opening 8 and a groove 9 are formed on said top plate 1C of said casing 1.

Fig. 1

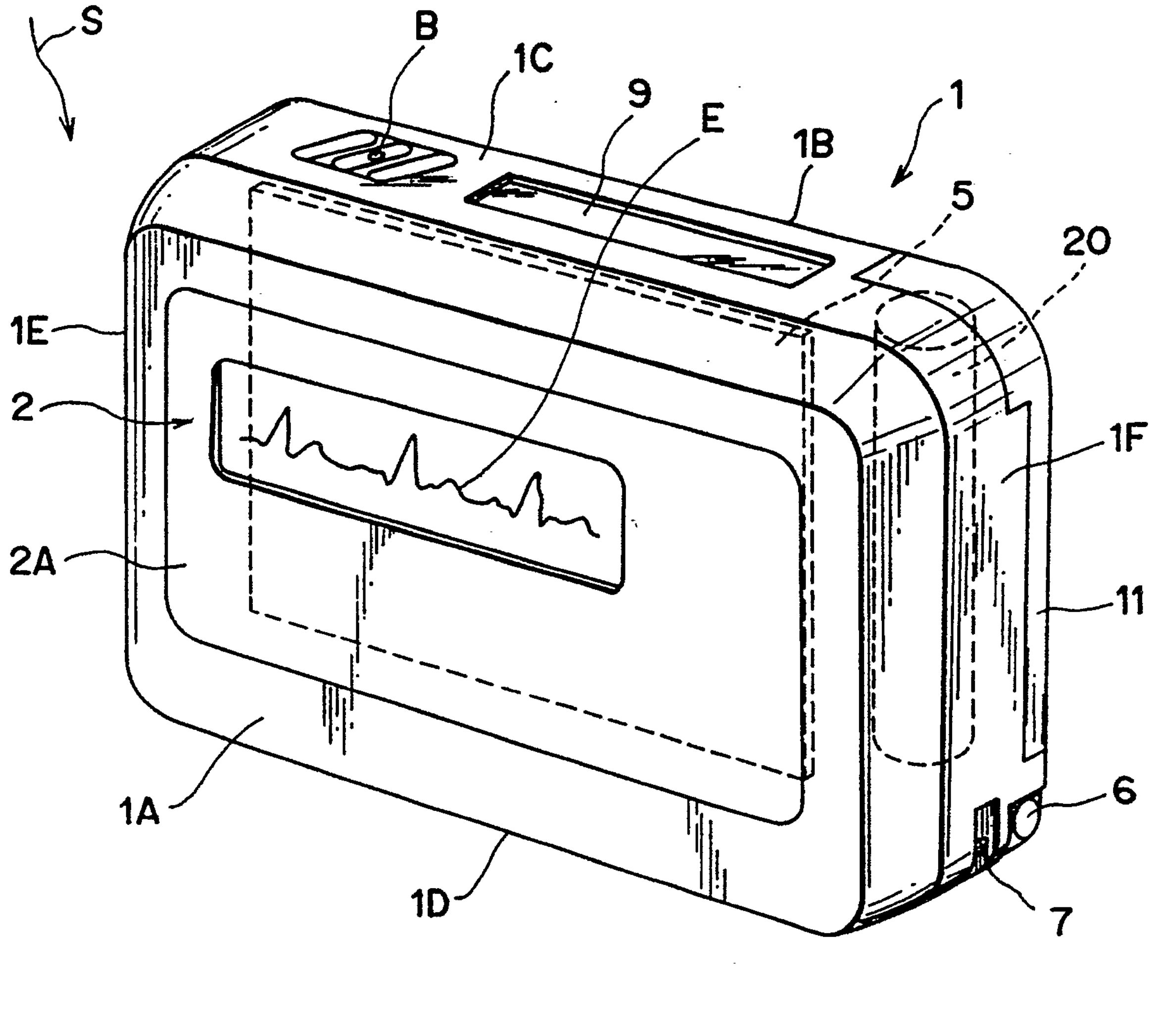

Fig. 2A

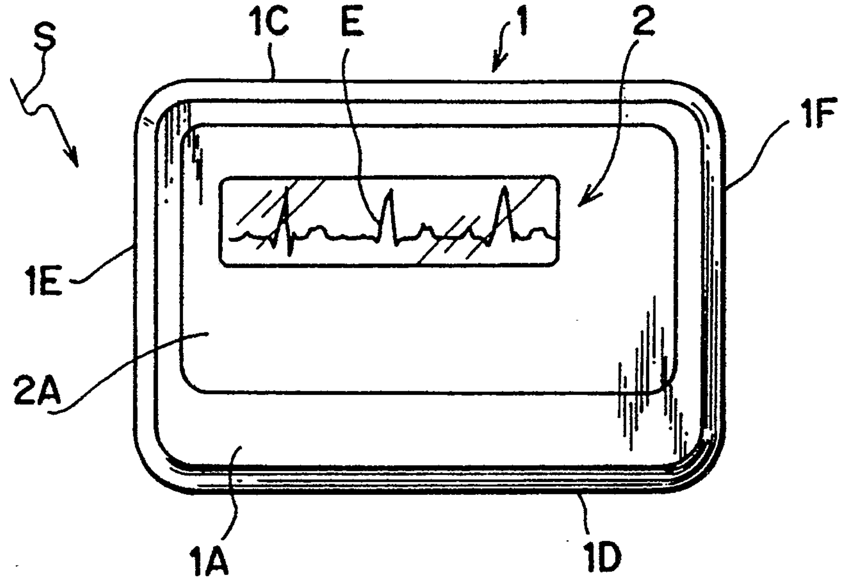


Fig. 2B

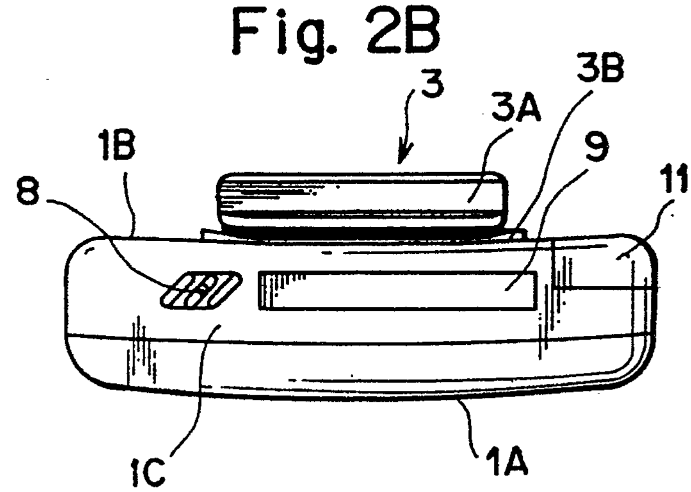

Fig. 2C

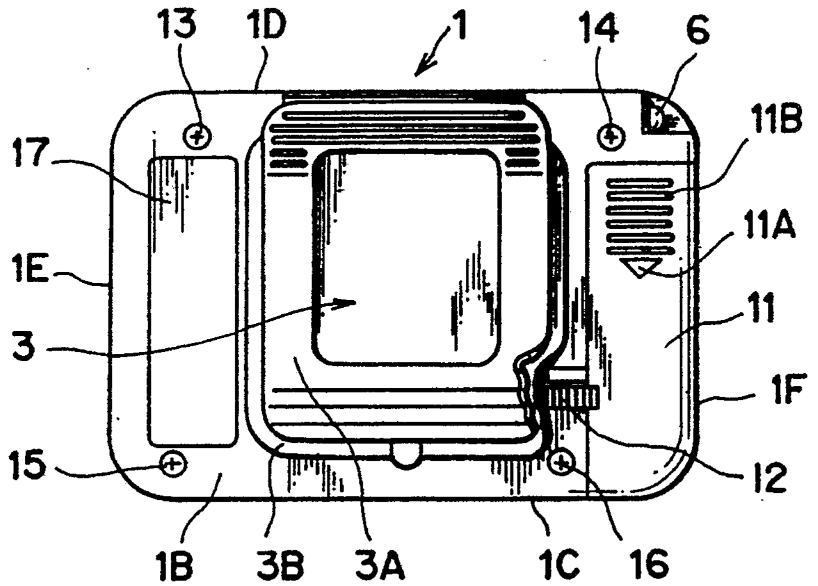


Fig. 2D

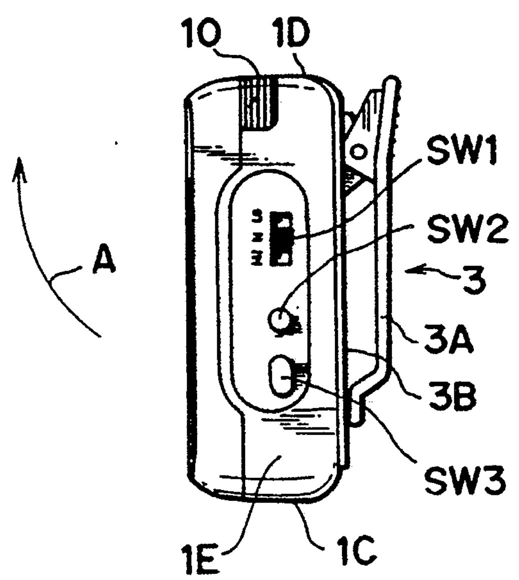


Fig. 2E

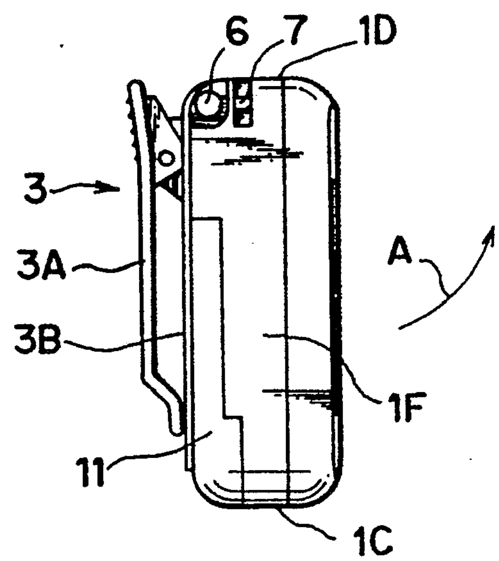

Fig. 3A

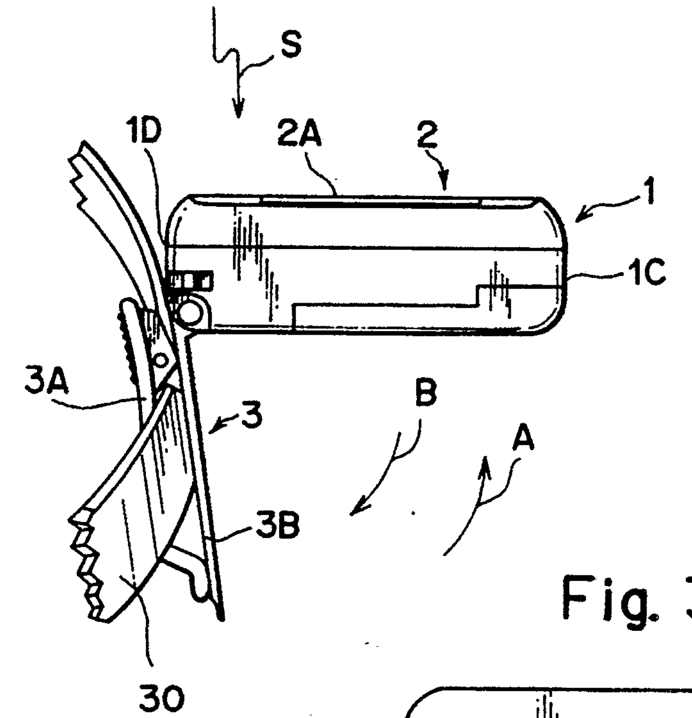


Fig. 3B

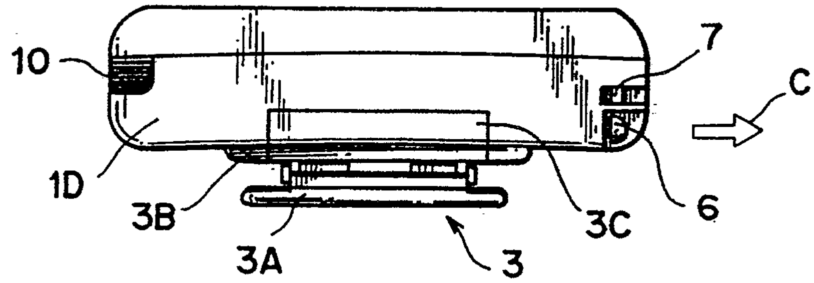


Fig. 3C

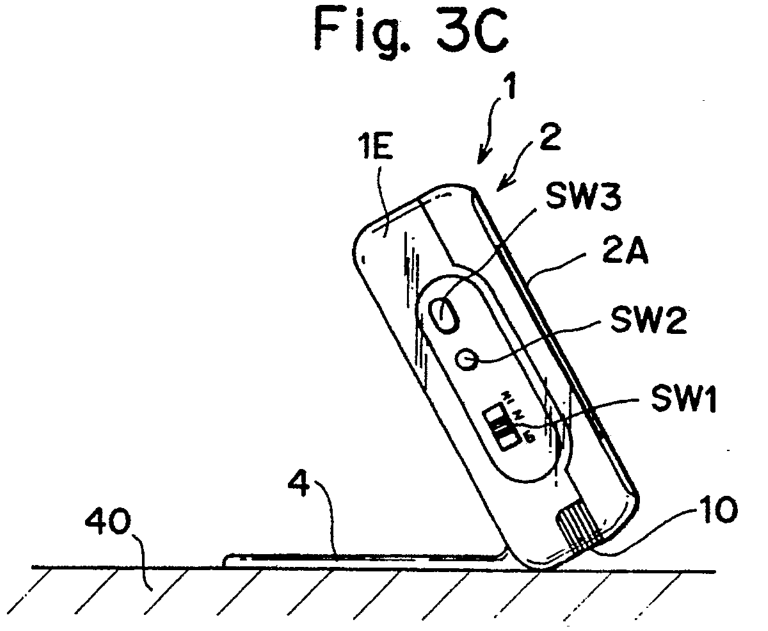

Fig. 4

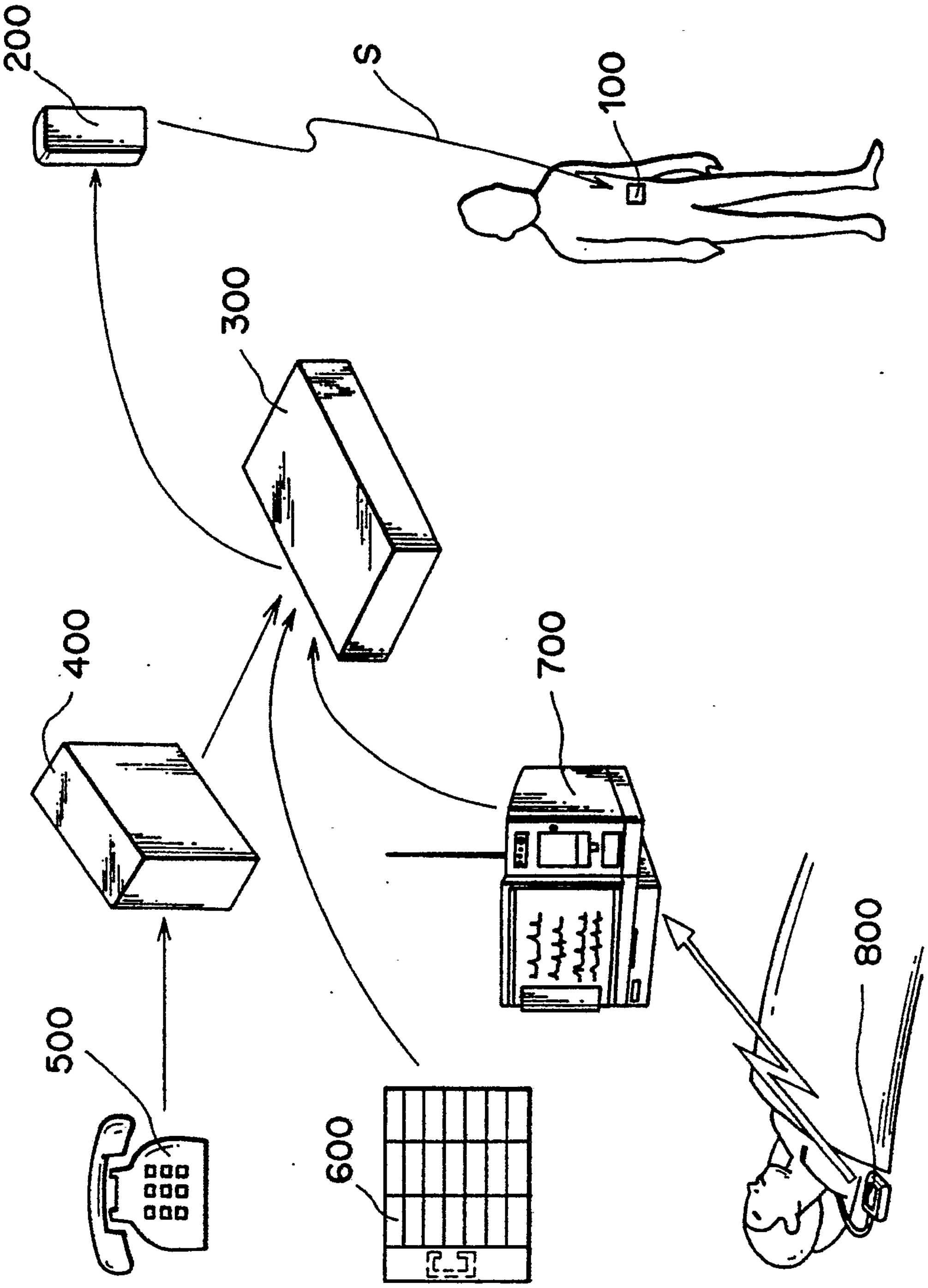

European Patent Office

# EUROPEAN SEARCH REPORT

Application Number

EP 90 10 1924

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-2 003 276 (A.E. KARZ)<br>* page 3, lines 20 - 79; figures 1-3 * * page 4, line 130 - page 5, line 120 * | 1,6,7 | A 61 B 5/00<br>A 61 B 5/0404 |
| Y | DE-A-2 107 072 (SIEMENS A.G.)<br>* page 2, lines 5 - 23; claims 1-4 * | 1 | |
| Y,A | EP-A-0 265 694 (H.-J. UHLEMANN)<br>* column 3, line 18 - column 4, line 31 * * column 6, line 47 - column 7, line 58 * * figures * | 1,2,3,6,8 | |
| A | PATENT ABSTRACTS OF JAPAN vol. 6, no. 66 (P-112)(944) 27 April 1982,<br>& JP-A-57 008418 (RICOH TOKEI K.K.) 16 January 1982,<br>* the whole document * | 1,2 | |
| | | | TECHNICAL FIELDS SEARCHED (Int. Cl.5) |
| | | | A 61 B<br>A 61 G |

The present search report has been drawn up for all claims

| Place of search | Date of completion of search | Examiner |
|---|---|---|
| The Hague | 21 December 90 | RIEB K.D. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document